# EUROPEAN PATENT APPLICATION

(11) **EP 1 983 046 A1**
(43) Date of publication of application: **22.10.2008**
(21) Application number: 07707949.9
(22) Date of filing: 02.02.2007
(51) Int. Cl.: C12N 15/00, C12N 15/09, C12Q 1/68

(54) **GENE GROUP APPLICABLE TO CANCER PROGNOSTICATION**

(30) Priority: 03.02.2006 JP 2006027735
(71) Applicant: MessengerScape Co. Ltd., Shibuya-ku Tokyo 151-0072 (JP); ORIENTAL YEAST CO., LTD., Tokyo 174-8505 (JP)
(72) Inventor: SAITO, Toshiyuki, Tokyo 1510072 (JP); MIKAMI, Yoji, Tokyo 1510072 (JP); KINUGASA, Masahiro, Nagahama-shi, Shiga 5260804 (JP); MORI, Kazuya, Nagahama-shi, Shiga 5260804 (JP); SUGIMOTO, Michiyo, Nagahama-shi, Shiga 5260804 (JP); UCHIDA, Koji, Nagahama-shi, Shiga 5260804 (JP)
(74) Representative: Graf von Stosch, Andreas
(86) International application number: PCT/JP2007/051800
(87) International publication number: WO 2007/088971

(57) **Abstract**

To provide a novel method for determining the risk of lymph node metastasis of breast cancer uses as an index the difference in the expression levels of marker genes between metastatic breast cancer tissue (or cell) and non-metastatic breast cancer tissue (or cell). The method involves (1) measuring the expression level of a gene having a base sequence in human metastatic breast cancer tissue (or cell), (2) measuring the expression level of the same gene in human non-metastatic breast cancer tissue (or cell), and (3) comparing the expression level of (1) with the expression level of (2) and determine the risk of lymph node metastasis of breast cancer based on the difference between the expression levels.

## Description

### Technical Field

The present invention relates to a novel method for determining the risk of lymph node metastasis of breast cancer. More specifically, the present invention relates to a method for determining the risk of lymph node metastasis of breast cancer that is based on comparison of the expression levels of marker genes having specific base sequences between metastatic breast cancer cells and non-metastatic breast cancer cells.

### Background Art

In Japan, the number of breast cancer patients is growing rapidly. The cancer is the most prevalent of all cancers in women. Estrogen, a female hormone, is considered a risk factor of breast cancer: women who have been exposed to estrogen for a longer period of time due to early menarche, late menopause, late age at first birth or nulliparity are more likely to develop breast cancer. Western-style high-fat diet and obesity are also associated with this type of cancer since estrogen is primarily produced in fat tissue in postmenopausal women. The changing lifestyles of Japanese women, such as their active participation in society, also contribute to the increase in the incidence of breast cancer.

Breast cancer is generally divided into three classes: non-invasive carcinomas, invasive carcinomas and Paget's disease of the breast. Most of the incidences of breast cancer that form lumps are invasive. There are common and special types of invasive breast cancers. The common types include scirrhoma, papillotubular carcinoma and solid-tubular carcinoma. The special types include mucinous carcinoma.

Because no blood test is available to specifically detect breast cancer, the detection of early breast cancers relies primarily on palpation and X-ray imaging. However, these techniques, even when used in combination, fail to detect as much as 20% of the cancer. In addition, diagnosis by X-ray imaging often requires specialists. The cytodiagnosis conducted before and during the surgical procedures can only be done by a pathologist and is often difficult due to the shortage of experienced pathologists and varying standards of the diagnosis. Thus, no subjective and simple technique for the detection/diagnosis of early breast cancers has ever existed to bridge the gap between detection and diagnosis of the disease. The PET analysis, a new diagnostic technique that can detect tumor tissue 1mm or less in diameter, requires large-scale facilities and is therefore not readily used for the detection of breast cancer.

Recent studies have shown that cancers are caused by anomalies in genes. For example, techniques have been proposed that detect cancer cells by making use of the fact that certain genes are expressed at different levels in a cancer tissue and a normal tissue (Patent Literatures 1 and 2).
[Patent Literature 1] Japanese Patent Application Laid-Open (JP-A) No. 2003-284594
[Patent Literature 2] Japanese Patent Application Laid-Open (JP-A) No. 2003-284596

### Disclosure of the Invention

Once lymph node-metastatic breast cancer has been removed by surgery, prognosis is predicted based on indices such as tumor size, nuclear pleomorphism of the removed cancer cells and of hormone receptor levels. Where necessary, adjuvant therapy is given to prevent metastasis to lymph nodes or the recurrence of cancer. The prediction of prognosis based on these presently available indices is not accurate enough, however, and more accurate indices for the prognosis of breast cancer patients are therefore needed to reduce the risk of recurrence and improve patients' quality of life by proper medication.

In view of the bove-described problems, the present inventors have conducted extensive studies and observed that certain marker genes are expressed at different levels in metastatic breast cancer cells or tissues and in non-metastatic breast cancer cells or tissues. The present inventors found that these marker genes could be used to determine the risk of lymph node metastasis of breast cancer and ultimately devised the present invention. Accordingly, the present invention provides the following measures to address the above-described problems.
(1) A method for determining the risk of lymph node metastasis of breast cancer, including using as an index the difference in the expression level of a marker gene between a metastatic breast cancer tissue (or cell) and a non-metastatic breast cancer tissue (or cell).
(2) The method according to (1) above, wherein the expression level of the marker gene is determined by the amount of mRNA of the gene.
(3) The method according to (1) or (2) above, wherein the marker gene is at least one selected from the group consisting of genes having base sequences of GenBank accession Nos. NM000903, NM006804, NM033547, CR611676, NM177967, NM152558, NM178167, NM003752, AK131568, CR592336, NM178507, NM002862, NM006913, NM005794, NM014164, NM000853 and a base sequence extending from 178882962bp to 178883181bp of chromosome 3, and homologs thereof.
(4) The method according to any one of (1) to (3) above, wherein the expression level of the marker gene in the metastatic breast cancer tissue (or cell) is equal to or higher than twice the expression level in the non-metastatic breast cancer tissue (cells), or equal to or lower than one-half the expression level in the non-metastatic breast cancer tissue.

The method of the present invention enables quick and simple determination of the risk of lymph node metastasis of breast cancer at the genetic level, thus providing an effective way to prevent metastasis of breast cancer.

### Brief Description of the Drawings

Fig. 1 is a diagram showing a comparison of the expression levels of a transcript (transcript 1) of a marker gene according to high-coverage gene expression profiling (HiCEP).
Fig. 2 is a diagram showing a comparison of the expression levels of a transcript (transcript 2) of another marker gene according to HiCEP.
Fig. 3 is a diagram showing a comparison of the expression levels of a transcript (transcript 3) of another marker gene according to HiCEP.
Fig. 4 is a diagram showing a comparison of the expression levels of a transcript (transcript 4) of another marker gene according to HiCEP.
Fig. 5 is a diagram showing a comparison of the expression levels of a transcript (transcript 5) of another marker gene according to HiCEP.
Fig. 6 is a diagram showing a comparison of the expression levels of a transcript (transcript 6) of another marker gene according to HiCEP.
Fig. 7 is a diagram showing a comparison of the expression levels of a transcript (transcript 7) of another marker gene according to HiCEP.
Fig. 8 is a diagram showing a comparison of the expression levels of a transcript (transcript 8) of another marker gene according to HiCEP.
Fig. 9 is a diagram showing a comparison of the expression levels of a transcript (transcript 9) of another marker gene according to HiCEP.
Fig. 10 is a diagram showing a comparison of the expression levels of a transcript (transcript 10) of another marker gene according to HiCEP.
Fig. 11 is a diagram showing a comparison of the expression levels of a transcript (transcript 11) of another marker gene according to HiCEP.
Fig. 12 is a diagram showing a comparison of the expression levels of a transcript (transcript 12) of another marker gene according to HiCEP.
Fig. 13 is a diagram showing a comparison of the expression levels of a transcript (transcript 13) of another marker gene according to HiCEP.
Fig. 14 is a diagram showing a comparison of the expression levels of a transcript (transcript 14) of another marker gene according to HiCEP.
Fig. 15 is a diagram showing a comparison of the expression levels of a transcript (transcript 15) of another marker gene according to HiCEP.
Fig. 16 is a diagram showing a comparison of the expression levels of a transcript (transcript 16) of another marker gene according to HiCEP.
Fig. 17 is a diagram showing a comparison of the expression levels of a transcript (transcript 17) of another marker gene according to HiCEP.

### Best Mode for Carrying Out the Invention

The present invention concerns a method for determining the risk of lymph node metastasis of breast cancer that uses as an index of the risk of metastasis the difference in the expression levels of specific marker genes between metastatic breast cancer cells or tissues and non-metastatic breast cancer cells or tissues. As used herein, the term "marker gene" refers to a gene that enables the determination of the risk of metastasis of breast cancer cells by comparing its expression levels between metastatic breast cancer cells or tissues and non-metastatic breast cancer cells or tissues.

The present invention also concerns a method for determining the risk of lymph node metastasis of breast cancer in which the expression levels of the marker genes are determined by the amounts of mRNA of the marker genes. More specifically, the present invention concerns a method for determining the risk of lymph node metastasis of breast cancer that involves extracting total RNA from cells obtained from metastatic and non-metastatic breast cancer tissues, and comparing the amounts of mRNA transcripts transcribed from the marker genes. Different techniques for gene expression analysis can be used to determine the amounts of mRNA of genes of interest, including comprehensive transcriptome analysis (high-coverage gene expression profiling, HiCEP), DNA microarrays, quantitative RT-PCR and northern hybridization. Gene expression analysis techniques that can determine the amounts of mRNA without extracting total RNA from cells, such as *in situ* hybridization, may also be used in the present invention. The above-described techniques may be used in combination to improve the accuracy of detection. The translated products of the genes of the present invention may also be quantified by, for example, determining the amounts of proteins coded by the genes. Proteins of interest can be quantified by using techniques such as immunological assays using antibodies specific for the proteins (such as ELISA, western blotting and RIA), two-dimensional electrophoresis and high-performance liquid chromatography (HPLC). Antibodies specific for the proteins coded by the genes of the present invention can be prepared by common techniques using the proteins coded by the genes as antigens.

HiCEP is one of the transcriptome analysis techniques and is characterized by its comprehensiveness and high sensitivity. The following is a brief overview of the technique (See Nucleic Acids Res., 2003, Vol. 31, No.16 e94 for more details): Using common techniques, total RNA is extracted and purified from tissue or cell samples. Double-stranded cDNA is synthesized from the total RNA using 5'-biotinylated oligo(dT) primers. The cDNA is then digested with a restriction enzyme MspI. Poly(A)-containing fragments are collected by avidin beads and 3'-adaptor is ligated to the MspI-digested ends of the collected fragments. The fragments are then digested with a restriction enzyme MseI and 3'-adapter is ligated to the MseI-digested ends. PCR primers are constructed by adding all possible combinations of two selected bases to the same adapter sequences as those ligated to 5' and 3' ends (16 5'-end primers and 16 3'-end primers with 5'-end primers fluorescent-labeled). Using these primers, 256 different quantitative PCRs are performed. The PCR products obtained for each primer pair are loaded on a fragment analyzer to obtain 256 electrophoresis profiles (gene expression profiles), each containing multiple fluorescence peaks, for a sample. The expression levels of transcripts can then be compared by comparing the fluorescence peaks among different samples.

The marker gene for use in the present invention may be any gene that is expressed at significantly different levels between metastatic breast cancer cells or tissues and non-metastatic breast cancer cells or tissues. For example, the marker gene may be at least one selected from the group consisting of genes having base sequences of GenBank accession Nos. NM000903, NM006804, NM033547, CR611676, NM177967, NM152558, NM178167, NM003752, AK131568, CR592336, NM178507, NM002862, NM006913, NM005794, NM014164 and NM000853 and a base sequence extending from 178882962bp to 178883181bp of chromosome 3, and homologs thereof.

Data stored in the GenBank database may contain the same gene registered by different researchers, at different times, in different fields and under different names or gene polymorphisms or splicing variants of the same gene registered as novel genes. Thus, different base sequences that can be considered to be originated from a single gene may be registered with different accession numbers. These base sequences are collectively referred to as "homologs." The term is used in the same context throughout this specification.

One characteristic feature of the method of the present invention for determining the risk of lymph node metastasis of breast cancer is the use of marker genes that are expressed at significantly different expression levels between metastatic breast cancer cells or tissues and non-metastatic breast cancer cells or tissues. The term "expression level" may refer to either the amount of mRNA transcribed from a marker gene or the amount of a protein translated from mRNA. With regard to the difference in the expression level of a marker gene between metastatic breast cancer cells or tissues and non-metastatic breast cancer cells or tissues, the ratio of the expression level of a marker gene in non-metastatic breast cancer cells or tissues to the expression level of the same gene in metastatic breast cancer cells or tissues is preferably in the range of 1.5 or higher or 2/3 or lower, and more preferably in the range of 2 or higher or 1/2 or lower. A marker gene does not serve as an accurate index of the risk of lymph node metastasis of breast cancer and is therefore not desirable when the ratio of its expression level in non-metastatic breast cancer cells or tissues to that in metastatic breast cancer cells or tissues is outside the above-described range.

While the method of the present invention can be applied to any type of breast cancer, including breast ductal carcinomas (such as papillotubular carcinoma, solid-tubular carcinoma and scirrhoma), lobular carcinomas, special-type carcinomas (such as mucinous carcinoma, medullary carcinoma and tubular carcinoma) and Paget's disease of the breast, it is preferably applied to scirrhoma, lobular carcinomas or solid-tubular carcinoma.

### Example

The present invention will now be described with reference to Examole, which is not intended to limit the scope of the invention in any way.

In this Example, the expression levels (RNA transcription levels) of different genes are compared between human metastatic breast cancer tissue and non-metastatic tissue using one of the gene expression analysis techniques known as high-coverage gene expression profiling technique (HiCEP), a known comprehensive, highly sensitive technique for transcriptome analysis (Nucleic Acids Res., 2003, vol.31(16), e94).

The breast cancer tissues used in Example were shown in Table 1. The tissues were collected from five stage II breast cancer patients (commercial products, all Caucasian, primary tumor, lymph node metastasis (2), no lymph node metastasis (3), all had stage II cancer based on TNM classification).

**Table 1**

| Samples | Metastasis | Tumor size | Age |
|---|---|---|---|
| #A | + | 12cm | 57 |
| #B | + | 2cm x 1.5cm x 1.5cm | 69 |
| #C | - | 2.5cm | 50 |
| #D | - | 4cm x 2cm x 1.7cm | 61 |
| #E | - | 6cm x 5.5cm x 4.5cm | 68 |

Total RNA was extracted from the samples by a common kit technique using RNeasy kit (Qiagen). 0.1µg of total RNA from each sample was used as template and reverse-transcribed using Super Script First Strand Synthesis System for RT-PCR (Invitrogen). The reverse transcript was incubated with DNA polymerase I (80 units), RNAase H (4 units, Invitrogen) and E. coli DNA ligase (40 units, Invitrogen) at 16°C for 2 hours. The resulting double-stranded DNA was incubated with restriction enzymes Mse I (40 units, New England Biolabs) and Msp I (50 units, TaKaRa Bio) at 37°C for 4 hours. Adaptor sequences were ligated to the ends of the resulting DNA fragments. Selective PCRs were performed using the adaptor-ligated DNA fragments as templates. The amplified products were analyzed by capillary electrophoresis. The waveform data were used to determine gene expression levels, compare the gene expression levels among the samples, and classify the genes into different expression patterns to obtain data for clustering (expression variation peaks).

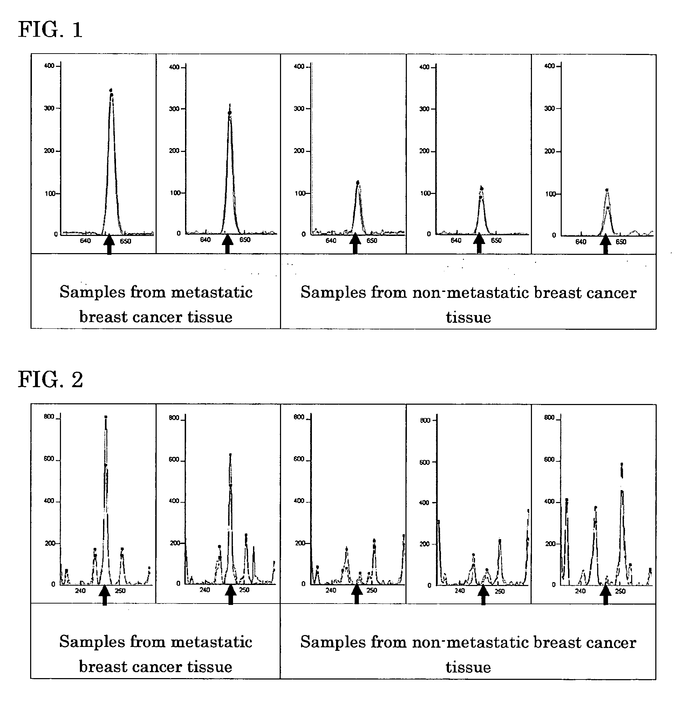

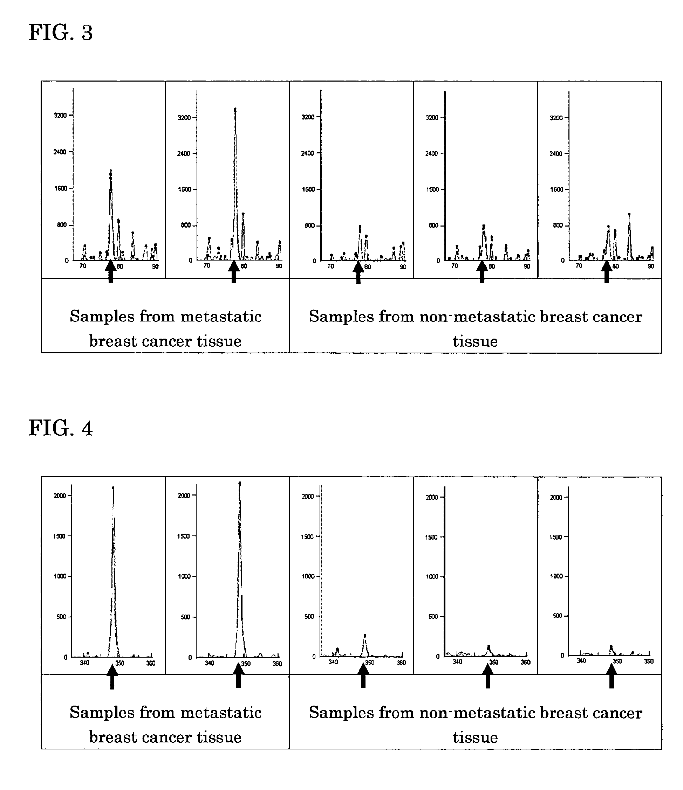

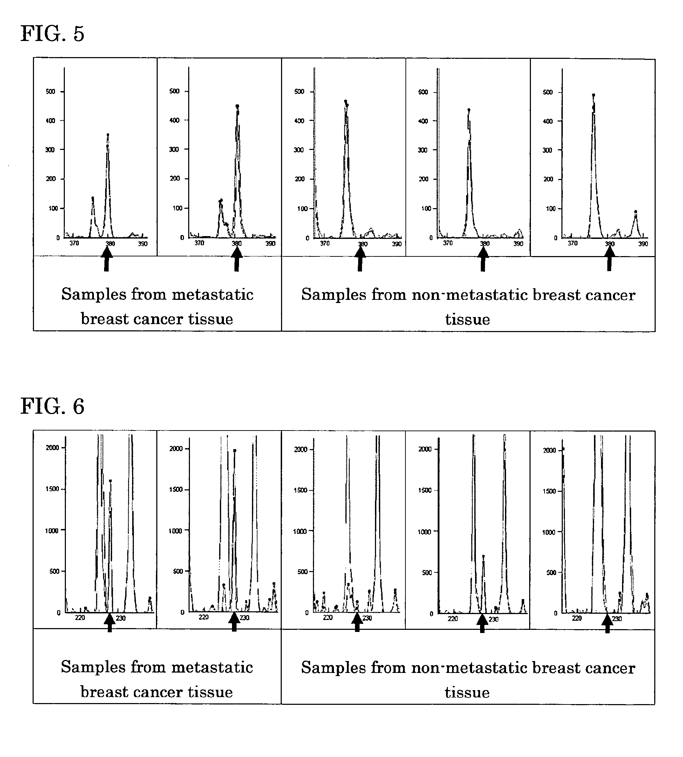

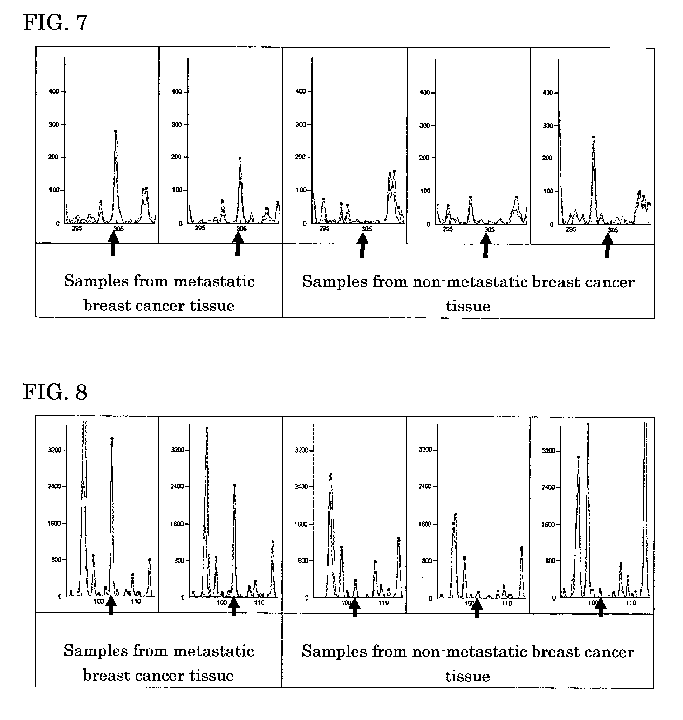

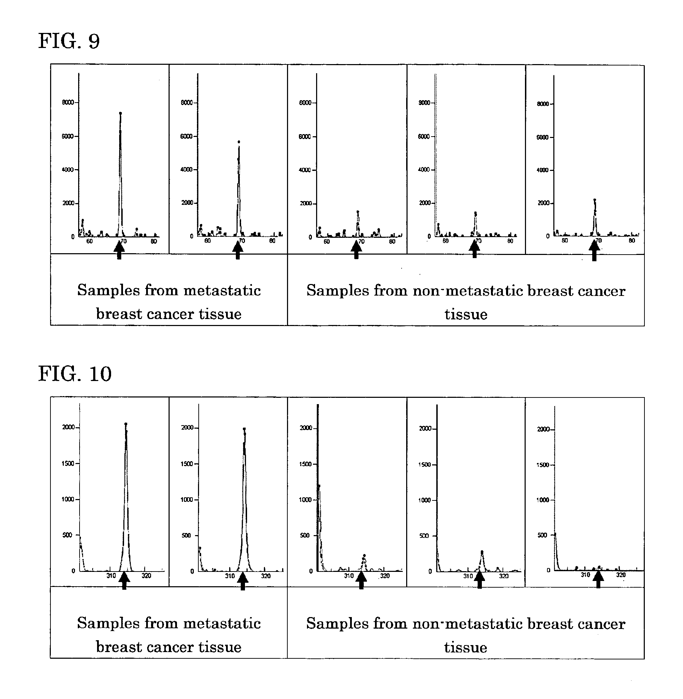

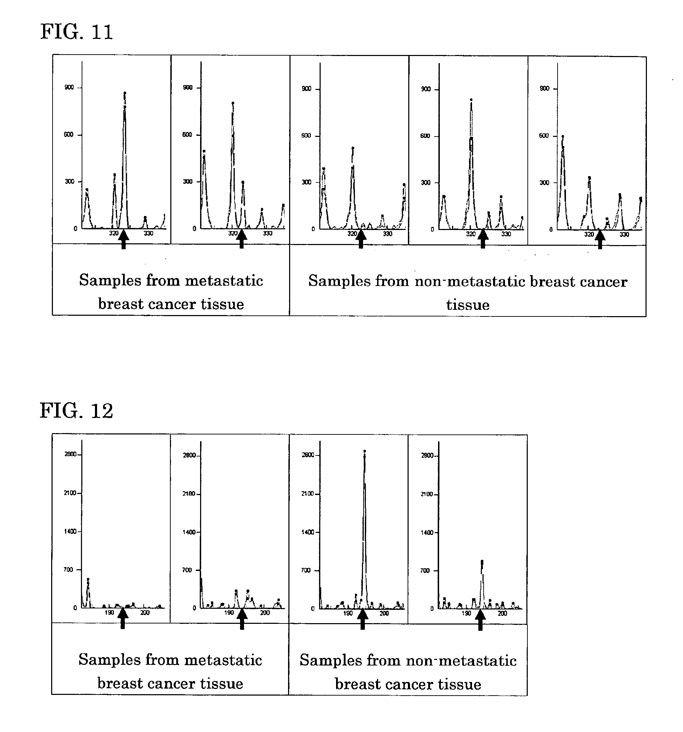

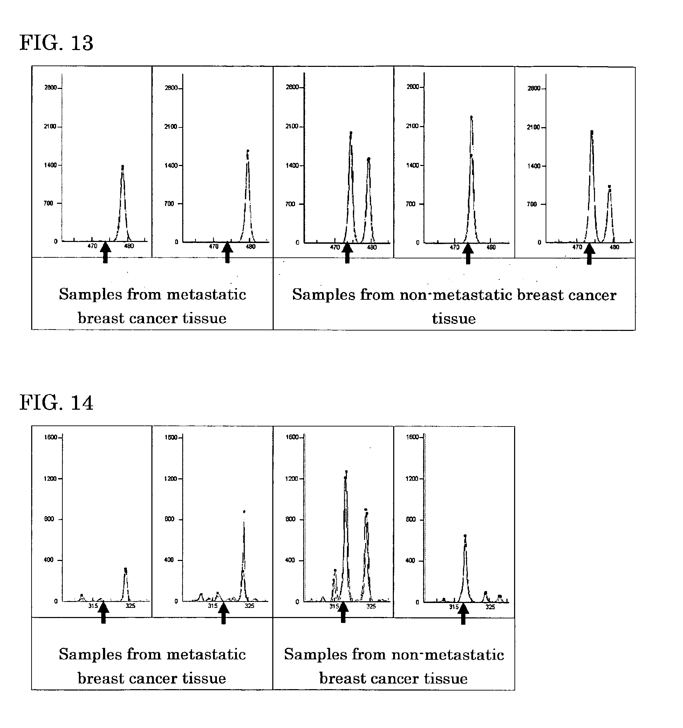

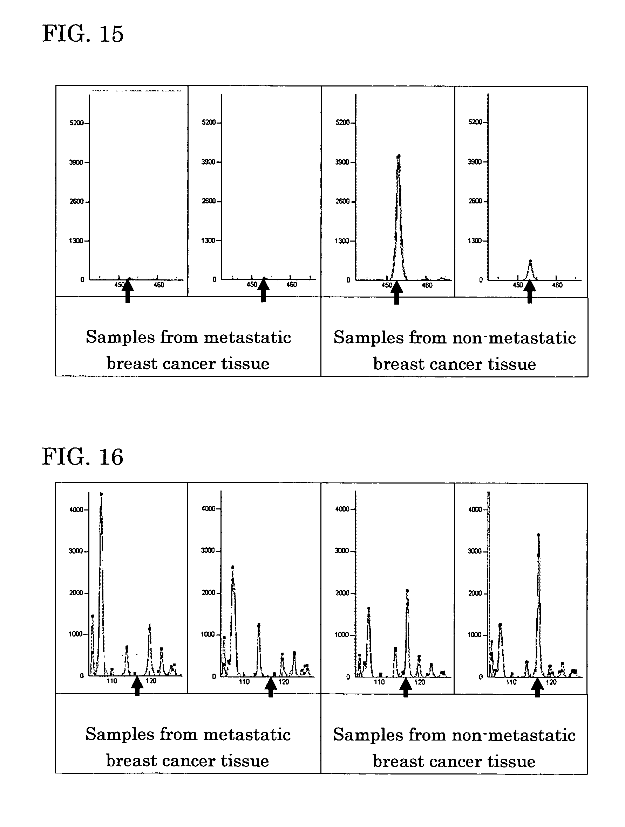

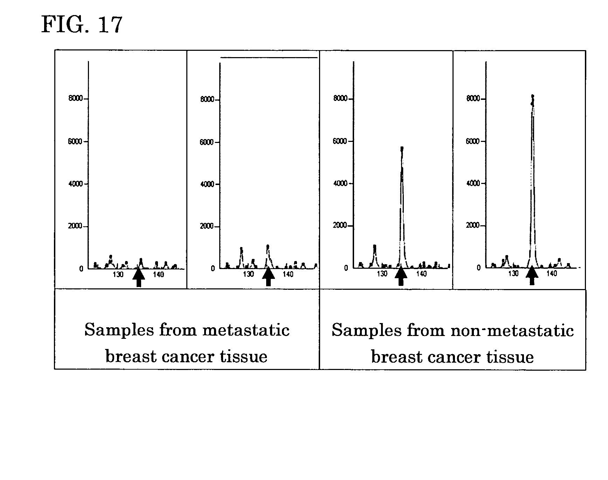

The results of the analysis shown in Table 2 and Figs. 1 through 17 demonstrate that the difference in the fluorescence peak intensity between samples obtained from patients with lymph node metastasis and samples obtained from patients with no metastasis was significant for each of the 17 marker gene transcripts. In this analysis, each sample was assayed in two replicates and the resulting fluorescence peaks were overlapped. Arrows indicate the peaks for the marker gene transcripts.

Specifically, Transcripts 1 through 11 (as numbered in Table 1) each show a significant fluorescence peak in each of the metastasis samples but show no expression peak or, if any, a peak intensity that is half or less of the peak intensity of the metastasis samples in each of the non-metastasis samples. Conversely, Transcripts 12 through 17 each show a significant fluorescence peak in each of the non-metastasis samples but show no expression peak or, if any, a peak intensity that is half or less of the peak intensity of the non-metastasis samples in each of the metastasis samples. These observations demonstrate that each of the 17 genes can serve as an index of the risk of breast cancer metastasis that allows the determination of the risk of metastasis based on their expression levels.

**Table 2. Transcripts as markers for breast cancer metastasis**

| Transcripts | Sequences | GenBank Accession No. | Annotation | Characteristics |
|---|---|---|---|---|
| #1 | Transcript sequence containing a base sequence from 68302490bp to 68317861bp of(-) strand of chromosome 16 | NM_000903 | NAD(P)H menadione oxidoreductase 1 | Experssion enhanced in metastatic breast cancer |
| #2 | Transcript sequence containing a base sequence from 178882962bp to 178883181bp of (+) strand | N/A | N/A | |
| #3 | Transcript sequence containing a base sequence from 35050592bp to 35050643bp of (+) strand of chromosome 17 | NM_006804 | steroidogenic acute regulatory protein related | |
| #4 | Transcript sequence containing a base sequence from 77267542bp to 77272569bp of (-) strand of chromosome 11 | NM_033547 | Homo sapiens hypothetical gene MGC16733 similar to CG12113 (MGC16733), mRNA. | |
| #5 | Transcript sequence containing a base sequence from 176665540bp to 176666255bp of (+) strand of chromosome 5 | CR611676 | Similar to Pxl9-like protein (25 kDa protein of relevant evolutionary and lymphoid interest) (PRELI) (CGI-106) (SBBI12) | |
| #6 | Transcript sequence containing a base sequence from 98835662bp to 98835862bp of (+) strand of chromosome 13 | NM_177967 | Phosphoglycerate dehydrogenase like 1 | |
| #7 | Transcript sequence containing a base sequence from 2426581bp to 2426860bp of(+) strand of chromosome 7 | NM_152558 | IQ motif containing E (IQCE) | |
| #8 | Transcript sequence containing a base sequence from 1987788 bp to 1987865 bp of (-) strand of chromosome 16 | NM_178167 | Zinc finger protein 598 | |
| #9 | Transcript sequence containing a base sequence from 228320033 bp to 28320077 bp of (-) strand of chromosome 16 | NM_003752 | Eukaryotic translation initiation factor 3, subunit 8, 110kDa | |
| #10 | Transcript sequence containing a base sequence from 35135544 bp to 35135831 bp of (+) strand of chromosome 17 | AK131568 | V-erb-b2 erythroblastic leukemia viral oncogene homolog 2, neuro/glioblastoma derived oncogene homolog (avian) | |
| #11 | Transcript sequence containing a base sequence from 35126382 bp to 35127393 bp of (+) strand of chromosome 17 | CR592336 | V-erb-b2 erythroblastic leukemia viral oncogene homolog 2, neuro/glioblastoma derived oncogene homolog (avian) | |
| #12 | Transcript sequence containing a base sequence from 119605680 bp to 119605847 bp of (+) strand of chromosome 11 | NM_178507 | NS5ATP13TP2 protein | Expression decreased in metastatic breast cancer |
| #13 | Transcript sequence containing a base sequence from 25226174 bp to 25226624 bp of (+) strand of chromosome 20 | NM_002862 | Phosphorylase, glycogen; brain | |
| #14 | Transcript sequence containing a base sequence from 32256007 bp to 32256297 bp of (+) strand of chromosome 6 | NM_006913 | Ring finger protein 5 | |
| #15 | Transcript sequence containing a base sequence from 23183541 bp to 23184510 bp of (-) strand of chromosome 14 | NM_005794 | Dehydrogenase/reduc tase (SDR family) member 2 | |
| #16 | Transcript sequence containing a base sequence from 40352503 bp to 40352595 bp of (+) strand of chromosome 19 | NM_014164 | FXYD domain containing ion transport regulator 5 | |
| #17 | Transcript sequence containing a base sequence from 22700873 bp to 22700983 bp of (+) strand of chromosome 22 | NM_000853 | Glutathione S-transferase theta 1 | |

### Industrial Applicability

Genes according to the present invention enable the highly sensitive and subjective, yet simple and quick determination of lymph node metastasis of breast cancer, a task that has never been achieved by any of the conventional techniques. The genes of the present invention therefore serve as markers for the prognosis of breast cancer.

## Claims

1. A method for determining the risk of lymph node metastasis of breast cancer, comprising: using as an index the difference in the expression level of a marker gene between a metastatic breast cancer tissue (or cell) and a non-metastatic breast cancer tissue (or cell).

2. The method according to claim 1, wherein the expression level of the marker gene is determined by the amount of mRNA of the gene.

3. The method according to claim 1 or 2, wherein the marker gene is at least one selected from the group consisting of genes having base sequences of GenBank accession Nos. NM000903, NM006804, NM033547, CR611676, NM177967, NM152558, NM178167, NM003752, AK131568, CR592336, NM178507, NM002862, NM006913, NM005794, NM014164, NM000853 and a base sequence extending from 178882962bp to 178883181bp of chromosome 3, and homologs thereof.

4. The method according to any of claims 1 to 3, wherein the expression level of the marker gene in the metastatic breast cancer tissue (or cell) is equal to or higher than twice the expression level in the non-metastatic breast cancer tissue (cells), or equal to or lower than one-half the expression level in the non-metastatic breast cancer tissue.
